# EUROPEAN PATENT APPLICATION

(11) **EP 4 353 081 A1**
(43) Date of publication of application: **17.04.2024**
(21) Application number: 22819915.4
(22) Date of filing: 30.03.2022
(51) Int. Cl.: A01N 37/02, A01N 37/06, A61K 8/36, A61K 8/92, A61K 8/99, A61Q 19/00

(54) **ANTIBACTERIAL COMPOSITION PRODUCTION METHOD, ANTIBACTERIAL COMPOSITION, ANTIBACTERIAL METHOD, ANTIBACTERIAL AGENT, COSMETIC, AND DERMATOLOGICAL TOPICAL AGENT**

(30) Priority: 07.06.2021 JP 2021095365
(71) Applicant: Ushio Denki Kabushiki Kaisha, Tokyo 100-8150 (JP)
(72) Inventor: KAWAI, Yuichi, Tokyo 100-8150 (JP)
(74) Representative: Gille Hrabal Partnerschaftsgesellschaft mbB Patentanwälte
(86) International application number: PCT/JP2022/015773
(87) International publication number: WO 2022/259727

(57) **Abstract**

Provided are an antibacterial composition, an antibacterial method, an antibacterial agent, a cosmetic, and a dermatological topical agent, that can selectively inhibit Staphylococcus aureus as compared with Staphylococcus epidermidis. Provided are an antibacterial composition production method including a step of producing a free fatty acid group by simple lipid and Staphylococcus epidermidis, an antibacterial composition obtained by the production method, an antibacterial composition containing a free fatty acid group derived from Staphylococcus epidermidis, an antibacterial method including a step of inhibiting Staphylococcus aureus by the antibacterial composition, an antibacterial agent containing the antibacterial composition, a cosmetic containing the antibacterial composition, and a dermatological topical agent containing the antibacterial composition.

## Description

### Technical Field

The present invention relates to an antibacterial composition production method, an antibacterial composition, an antibacterial method, an antibacterial agent, a cosmetic, and a dermatological topical agent.

### Background Art

It has been reported that Staphylococcus epidermidis, which accounts for a large percentage in indigenous bacteria on the skin, produces a substance for suppressing the activity of other bacteria, such as glycerol, free fatty acids, and antibacterial peptide, from sebum as a raw material and provides a part of immunological mechanism of a surface of the skin.

Staphylococcus epidermidis on the skin surface has no toxicity. Such properties are useful for maintenance of the health of the skin, and Staphylococcus epidermidis is representative of useful bacteria, for example, in the field of cosmetic, and the like.

On the other hand, Staphylococcus aureus is a bacterium that is known to be a food poisoning bacterium and has strong toxicity, and causes skin trouble including atopic dermatitis on the skin surface. Therefore, Staphylococcus aureus is representative of harmful bacteria, for example, in the field of cosmetic, and the like.

A healthy skin surface is kept slightly acidic by a free fatty acid produced by Staphylococcus epidermidis, which suppresses the activity of Staphylococcus aureus that prefers a slightly alkaline environment.

Thus, on the skin surface, certain bacteria limit the activity of other bacteria to keep a balance of the bacteria, which maintains a hygienic environment. For routine skin care, it is important to suppress the activity of the harmful bacteria by a simple method without impairing the balance.

As a method for removing the harmful bacteria, administration of an antibiotic and disinfection with rubbing alcohol are well known. However, the former requires identification of causative bacteria and monitoring from the viewpoint of dramatic effects, appearance of resistant bacteria, and the like. This shows that the former is not simple. The latter can be simply used, but useful bacteria are also killed. Further, the latter deteriorates the barrier function of the skin itself due to loss of oils on the skin surface. Therefore, both the former and the latter have problems with the routine skin care.

A technique for mixing a substance having a selective antibacterial action on the harmful bacteria (Staphylococcus aureus) in a cosmetic or the like is known. As the substance having a selective antibacterial action, a fatty acid is known. PTLs 1 to 4 describe techniques using fatty acids as the substance having a selective antibacterial action.

PTL 1 describes that an antibacterial agent containing as an active ingredient a fatty acid derivative represented by a specific chemical formula or a salt thereof can prevent or treat Propionibacterium acnes infectious skin diseases, such as acne and seborrheic dermatitis, or prevent or treat skin diseases, such as atopic dermatitis, cutaneous pruritus, and contact dermatitis, while the distribution of indigenous bacteria on the skin is kept in a healthy state.

PTL 2 describes a skin improving agent containing as an active ingredient that inhibits the proliferation of Staphylococcus aureus and proliferates Staphylococcus epidermidis at least one selected from the group consisting of a branched saturated fatty acid having 10 to 18 carbon atoms on a main chain, 6-hexadecenoic acid, palmitoleic acid, linoleic acid, α-linolenic acid, a linoleic acid methyl ester, an α-linolenic acid methyl ester, and arachidonic acid.

PTL 3 describes a cosmetic and/or dermatological topical composition, especially useful for the prevention and/or treatment of sensitive and/or dry skin, containing at least an effective amount of at least one type of probiotic microorganism and/or a fraction or metabolite thereof in combination with an effective amount of at least one type of polyunsaturated fatty acid and/or polyunsaturated fatty acid ester and/or a salt or derivative thereof in a physiologically-acceptable medium.

PTL 4 describes that a skin cosmetic containing (a) 0.01 to 20 wt% of at least one selected from isostearic acid, 12-methyl tetradecanoic acid, 13-methyl tetradecanoic acid, 6-hexadecenoic acid, linoleic acid, α-linolenic acid, a linoleic acid methyl ester, an α-linolenic acid methyl ester, and arachidonic acid, and (b) at least one selected from the group consisting of petrolatum, lanolin, cholesterol, bleached beeswax, paraffin wax, candelilla wax, rice bran wax, cetyl myristate, ceresin, microcrystalline wax, ozokerite, plastibase, simple ointment, absorption ointment, hydrous lanolin, macrogol ointment, and gel base that are in a paste form or a solid form at normal temperature achieves actions that prevent or improve drying of the skin and prevent or improve a rough skin, and is suitable for improvement of a skin condition or prevention of a rough skin, especially in skin diseases, such as atopic dermatitis.

### Citation List

### Patent Literature

PTL 1: JP 2012-144453 A
PTL 2: JP 2001-172176 A
PTL 3: JP 2009-503042 A
PTL 4: JP 2006-282650 A

### Summary of Invention

### Technical Problem

The present invention provides an antibacterial composition, an antibacterial method, an antibacterial agent, a cosmetic, and a dermatological topical agent, that can selectively inhibit Staphylococcus aureus as compared with Staphylococcus epidermidis.

### Solution to Problem

The present inventors have intensively studied and as a result found that a free fatty acid group produced by simple lipid and Staphylococcus epidermidis selectively inhibits Staphylococcus aureus as compared with Staphylococcus epidermidis. Thus, the present invention has been completed.

Specifically, the present invention provides an antibacterial composition production method, an antibacterial composition, an antibacterial method, an antibacterial agent, a cosmetic, and a dermatological topical agent described below.

[1] A method for producing an antibacterial composition including: a step of producing a free fatty acid group by simple lipid and Staphylococcus epidermidis.
[2] The method for producing an antibacterial composition according to [1], in which the simple lipid contains at least one selected from the group consisting of oil and fat and wax.
[3] The method for producing an antibacterial composition according to [1] or [2], in which the simple lipid contains a saturated fatty acid having 8 or more and 18 or less carbon atoms in an amount of 10 mass% or more relative to 100 mass% of a total amount of a fatty acid moiety constituting the simple lipid.
[4] The method for producing an antibacterial composition according to any of [1] to [3], in which the simple lipid contains at least one type of oil and fat selected from the group consisting of coconut oil, palm kernel oil, palm olein, palm oil, linseed oil, grape oil, soybean oil, sunflower oil, corn oil, cottonseed oil, sesame oil, rice oil, peanut oil, and olive oil.
[5] The method for producing an antibacterial composition according to any of [1] to [4], in which a content of the free fatty acid having 8 or more and 18 or less carbon atoms in the antibacterial composition is 5 mass% or more and 50 mass% or less relative to 100 mass% of a total amount of the antibacterial composition.
[6] An antibacterial composition produced by the production method according to any of [1] to [5].
[7] An antibacterial composition containing a free fatty acid group derived from Staphylococcus epidermidis.
[8] The antibacterial composition according to [7], in which a content of the free fatty acid having 8 or more and 18 or less carbon atoms in the antibacterial composition is 5 mass% or more and 50 mass% or less relative to 100 mass% of a total amount of the antibacterial composition.
[9] An antibacterial method including: a step of inhibiting Staphylococcus aureus by the antibacterial composition according to any of [6] to [8].
[10] An antibacterial agent containing: the antibacterial composition according to any of [6] to [8].
[11] A cosmetic containing: the antibacterial composition according to any of [6] to [8].
[12] A dermatological topical agent containing: the antibacterial composition according to any of [6] to [8].

### Advantageous Effects of Invention

The present invention can provide an antibacterial composition, an antibacterial method, an antibacterial agent, a cosmetic, and a dermatological topical agent, that can selectively inhibit Staphylococcus aureus as compared with Staphylococcus epidermidis. Brief Description of Drawings

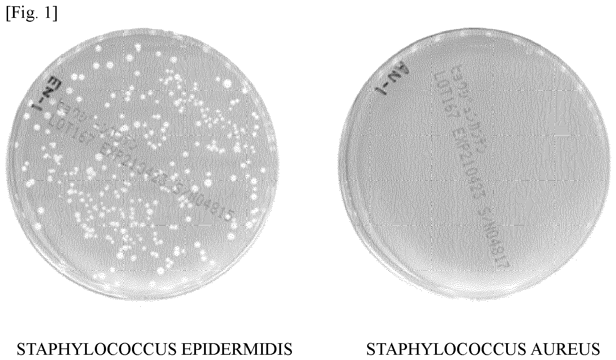

Fig. 1 is a view showing photographs of a Staphylococcus epidermidis test area and a Staphylococcus aureus test area obtained in evaluation of Example 1.

### Description of Embodiments

### [Antibacterial Composition Production Method]

An antibacterial composition production method of the present invention includes a step (A) of producing a free fatty acid group by simple lipid and Staphylococcus epidermidis. In the present description, the free fatty acid group represents a composition of free fatty acid consisting of two or more types of free fatty acids.

According to the antibacterial composition production method of the present invention, an antibacterial composition that can selectively inhibit Staphylococcus aureus as compared with Staphylococcus epidermidis can be obtained.

Although the reason that the antibacterial composition produced by the production method of the present invention can selectively inhibit Staphylococcus aureus as compared with Staphylococcus epidermidis is not clear, the reason is considered as follows.

Fatty acids have a structure similar to the structure of a cell membrane component covering a surface of bacteria. Therefore, fatty acids have an action of melting into the cell membrane of the bacteria, changing the properties of the cell membrane, and making it difficult to sustain life of the bacteria, to kill the bacteria.

The component constituting the cell membrane of the bacteria varies depending on the type thereof. Therefore, a degree of melting into the cell membrane of each bacterium of fatty acids varies depending on the type of the fatty acids. There are a large numbers of fatty acids. From the fatty acids, a fatty acid that melts into a cell membrane of Staphylococcus aureus to be killed needs to be selected. However, in this selection method, not only the activity of Staphylococcus aureus is suppressed, but also the activity of Staphylococcus epidermidis may be inhibited.

On the other hand, since the free fatty acid group produced by the antibacterial composition production method of the present invention is a substance derived from Staphylococcus epidermidis, the free fatty acid group is considered not to suppress the activity of Staphylococcus epidermidis.

The reasons described above show that the antibacterial composition produced by the production method of the present invention can selectively inhibit Staphylococcus aureus as compared with Staphylococcus epidermidis.

Since Staphylococcus epidermidis is indigenous bacteria on the skin, a substance produced by Staphylococcus epidermidis is considered to have high safety. Therefore, the antibacterial composition of the present invention containing the free fatty acid group derived from Staphylococcus epidermidis is considered to have favorable safety.

### <Step (A)>

The step (A) is a step of producing the free fatty acid group by simple lipid and Staphylococcus epidermidis.

In the step (A), for example, Staphylococcus epidermidis is cultivated with simple lipid, to produce the free fatty acid group. At that time, a plurality of triacylglycerols contained in simple lipid are hydrolyzed by an enzyme secreted from Staphylococcus epidermidis, such as lipase, to produce the free fatty acid group.

Staphylococcus epidermidis used in the step (A) is not particularly limited as long as it is a strain that belongs to Staphylococcus epidermidis and is capable of producing the free fatty acid group from simple lipid. Preferable examples of the strain include ATCC12228 and ATCC14990.

The simple lipid used in the step (A) refers to an ester of an alcohol and a fatty acid. In an alcohol moiety, for example, a linear alcohol, a glycerol, a sterol, or the like is used, and in a fatty acid moiety, for example, a variety of saturated fatty acids or unsaturated fatty acids is used.

From the viewpoint of more effectively producing the free fatty acid group by Staphylococcus epidermidis, the simple lipid used in the step (A) preferably contains at least one selected from the group consisting of oil and fat (triacylglycerol) and wax (ester oil). From the viewpoint of improving oxidation resistance, the simple lipid is preferably simple lipid having a fatty acid moiety containing a saturated fatty acid. From the viewpoint of improving oxidation resistance and further improving antibacterial properties against Staphylococcus aureus, the simple lipid is preferably simple lipid containing a saturated fatty acid having 8 or more and 18 or less carbon atoms.

From the viewpoint of further improving antibacterial properties against Staphylococcus aureus and further improving oxidation resistance, the simple lipid contains a saturated fatty acid having 8 or more and 18 or less carbon atoms in an amount of preferably 10 mass% or more, more preferably 30 mass% or more, further preferably 50 mass% or more, still further preferably 70 mass% or more, still further preferably 80 mass% or more, and still further preferably 85 mass% or more, and preferably 100 mass% or less, relative to 100 mass% of a total amount of the fatty acid moiety constituting the simple lipid.

From the viewpoint of further improving antibacterial properties against Staphylococcus aureus, the number of carbon atoms of the fatty acid moiety constituting the simple lipid is preferably 8 or more, and more preferably 10 or more, and from the same viewpoint, the number is preferably 18 or less, more preferably 16 or less, and further preferably 14 or less.

From the viewpoint of further improving antibacterial properties against Staphylococcus aureus, the fatty acid constituting the simple lipid preferably contains at least one selected from the group consisting of caprylic acid, capric acid, lauric acid, myristic acid, palmitic acid, palmitoleic acid, stearic acid, oleic acid, linoleic acid, and linolenic acid. From the viewpoint of further improving antibacterial properties against Staphylococcus aureus and further improving oxidation resistance, the fatty acid more preferably contains at least one selected from the group consisting of caprylic acid, capric acid, lauric acid, myristic acid, palmitic acid, and stearic acid, further preferably contains at least one selected from the group consisting of lauric acid, myristic acid, and palmitic acid, and still further preferably contains lauric acid, myristic acid, and palmitic acid. Among these fatty acids, lauric acid has excellent antibacterial properties against Staphylococcus aureus and exhibits antibacterial properties against Propionibacterium acnes (bacteria causing acne). Therefore, the fatty acid constituting the simple lipid further preferably contains lauric acid.

From the viewpoint of further improving antibacterial properties against Staphylococcus aureus and further improving oxidation resistance, the simple lipid according to the embodiment preferably contains at least one type of oil and fat selected from the group consisting of coconut oil, palm kernel oil, palm olein, palm oil, linseed oil, grape oil, soybean oil, sunflower oil, corn oil, cottonseed oil, sesame oil, rice oil, peanut oil, and olive oil, more preferably contains at least one type of oil and fat selected from the group consisting of coconut oil, palm kernel oil, palm olein, and palm oil, further preferably contains at least one type of oil and fat selected from the group consisting of coconut oil and palm kernel oil, is still further preferably at least one type of oil and fat selected from the group consisting of coconut oil and palm kernel oil, and is still further preferably coconut oil.

Herein, coconut oil and palm kernel oil are preferred since they contain a saturated fatty acid at a high ratio and have favorable oxidation resistance. Coconut oil and palm kernel oil are unlikely to be altered in properties during storage due to favorable oxidation resistance, and have an advantage that storage management is easy.

In the antibacterial composition production method according to the present invention, the fatty acid composition of the free fatty acid group obtained can be changed by changing the type of the simple lipid used in the step (A). Thus, the antibacterial composition can be controlled so as to be suitable for a variety of Staphylococcus aureus.

In the step (A), a culture medium used during cultivation of Staphylococcus epidermidis with the simple lipid is not particularly limited, and a publicly known culture medium usable during cultivation of Staphylococcus epidermidis or a modified culture medium can be used.

In the step (A), a method for cultivating Staphylococcus epidermidis with the simple lipid to produce the free fatty acid group is not particularly limited, and examples thereof include as follows.

The simple lipid is added to a culture medium, a trace amount of surfactant is added to a solution to sufficiently bring the simple lipid into contact with the culture medium, and Staphylococcus epidermidis is inoculated with vigorous stirring such that an aqueous layer and an oil layer are mixed to obtain an emulsion liquid. Subsequently, a homogeneous emulsification state is held with stirring. While the whole container is kept, for example, at 10°C to 45°C, cultivation is performed for a predetermined period of time until a reaction (fermentation) sufficiently proceeds. After completion of the cultivation, an autoclave treatment is performed until the solution reaches a cloud point, resulting in demulsification at the same time of sterilization, to separate the solution into an aqueous layer and an oil layer. Subsequently, the solution is cooled, and the separated oil layer is coagulated and taken out. The taken oil layer is heated and melts, to obtain a white cloudy oil. This oil is separated by centrifugation into an aqueous layer containing the culture medium, a contaminant layer containing dead bacteria, and a transparent oil layer. The transparent oily ingredient obtained contains the free fatty acid group.

### <Free Fatty Acid Group>

From the viewpoint of further improving antibacterial properties against Staphylococcus aureus and further improving oxidation resistance, the content of the free fatty acid having 8 or more and 18 or less carbon atoms in the antibacterial composition according to the present invention is preferably 5 mass% or more, more preferably 8 mass% or more, further preferably 10 mass% or more, and still further preferably 12 mass% or more, relative to 100 mass% of a total amount of the antibacterial composition. From the viewpoint of further improving selective antibacterial properties for Staphylococcus aureus as compared with Staphylococcus epidermidis, the content is more preferably 50 mass% or less, further preferably 30 mass% or less, still further preferably 20 mass% or less, and still further preferably 18 mass% or less.

From the viewpoint of further improving antibacterial properties against Staphylococcus aureus and further improving oxidation resistance, the content of the free fatty acid having 10 or more and 16 or less carbon atoms in the antibacterial composition according to the present invention is preferably 4 mass% or more, more preferably 8 mass% or more, further preferably 10 mass% or more, and still further preferably 12 mass% or more, relative to 100 mass% of a total amount of the antibacterial composition. From the viewpoint of further improving selective antibacterial properties for Staphylococcus aureus as compared with Staphylococcus epidermidis, the content is more preferably 50 mass% or less, further preferably 30 mass% or less, still further preferably 20 mass% or less, still further preferably 18 mass% or less, and still further preferably 16 mass% or less.

From the viewpoint of further improving antibacterial properties against Staphylococcus aureus and further improving oxidation resistance, the content of caprylic acid, capric acid, lauric acid, myristic acid, and palmitic acid in the antibacterial composition according to the present invention is preferably 4 mass% or more, more preferably 8 mass% or more, further preferably 10 mass% or more, and still further preferably 12 mass% or more, relative to 100 mass% of a total amount of the antibacterial composition. From the viewpoint of further improving selective antibacterial properties for Staphylococcus aureus as compared with Staphylococcus epidermidis, the content is more preferably 50 mass% or less, further preferably 30 mass% or less, still further preferably 20 mass% or less, still further preferably 18 mass% or less, and still further preferably 16 mass% or less.

From the viewpoint of further improving antibacterial properties against Staphylococcus aureus and further improving oxidation resistance, the content of lauric acid, myristic acid, and palmitic acid in the antibacterial composition according to the present invention is preferably 3 mass% or more, more preferably 5 mass% or more, further preferably 8 mass% or more, and still further preferably 10 mass% or more, relative to 100 mass% of a total amount of the antibacterial composition. From the viewpoint of further improving selective antibacterial properties for Staphylococcus aureus as compared with Staphylococcus epidermidis, the content is more preferably 30 mass% or less, further preferably 20 mass% or less, still further preferably 18 mass% or less, still further preferably 15 mass% or less, and still further preferably 14 mass% or less.

From the viewpoint of further improving selective antibacterial properties for Staphylococcus aureus as compared with Staphylococcus epidermidis, further improving antibacterial properties against Staphylococcus aureus, and further improving oxidation resistance, the free fatty acid group preferably contains three or more selected from the group consisting of caprylic acid, capric acid, lauric acid, myristic acid, palmitic acid, stearic acid, oleic acid, and linoleic acid, more preferably five or more selected from the group, further preferably six or more selected from the group, still further preferably seven or more selected from the group, and still further preferably all eight of the group.

Among the free fatty acids, lauric acid has excellent antibacterial properties against Staphylococcus aureus and exhibits antibacterial properties against Propionibacterium acnes (bacteria causing acne). Therefore, the free fatty acid group according to the present invention further preferably contains lauric acid.

### [Antibacterial Composition]

The antibacterial composition of the present invention is a composition obtained by the antibacterial composition production method according to the present invention or a composition containing a free fatty acid group derived from Staphylococcus epidermidis.

Herein, the free fatty acid group derived from Staphylococcus epidermidis means a free fatty acid group produced by Staphylococcus epidermidis or a free fatty acid group obtained by a reaction of the simple lipid with Staphylococcus epidermidis.

According to the antibacterial composition of the present invention, Staphylococcus aureus can be selectively inhibited as compared with Staphylococcus epidermidis. Although the reason that the antibacterial composition of the present invention exerts the effects of the present invention is not clear, the reason is considered to be the same as the reason that the antibacterial composition production method according to the present invention exerts the effects of the present invention.

Since Staphylococcus epidermidis is indigenous bacteria on the skin, a substance produced by Staphylococcus epidermidis is considered to have high safety. Therefore, the antibacterial composition of the present invention containing the free fatty acid group derived from Staphylococcus epidermidis is considered to have favorable safety.

A suitable aspect of the free fatty acid group contained in the antibacterial composition according to the present invention is the same as the free fatty acid group in the antibacterial composition production method according to the present invention.

From the viewpoint of further improving selective antibacterial properties for Staphylococcus aureus as compared with Staphylococcus epidermidis and further improving antibacterial properties against Staphylococcus aureus, the content of the free fatty acid group derived from Staphylococcus epidermidis in the antibacterial composition according to the present invention is preferably 50 mass% or more, more preferably 70 mass% or more, further preferably 80 mass% or more, still further preferably 90 mass% or more, and still further preferably 95 mass% or more, and preferably 100 mass% or less, relative to 100 mass% of a total amount of the free fatty acid contained in the antibacterial composition according to the present invention.

### [Antibacterial Method]

An antibacterial method of the present invention includes a step of inhibiting Staphylococcus aureus by the antibacterial composition according to the present invention. According to the antibacterial method of the present invention, Staphylococcus aureus can be selectively inhibited as compared with Staphylococcus epidermidis.

Herein, the antibacterial method according to the present invention can be performed, for example, by administering the antibacterial composition according to the present invention to a surface of a subject. The surface of the subject is preferably the skin.

Examples of a method for administering the antibacterial composition according to the present invention to the surface of the subject include a method for applying the antibacterial composition to the surface of the subject and a method for spraying the antibacterial composition onto the surface of the subject.

### [Antibacterial Agent]

The antibacterial composition according to the present invention can be suitably used for an antibacterial agent.

Specifically, an antibacterial agent of the present invention contains the antibacterial composition according to the present invention.

In the present description, the "antibacterial agent" refers to a chemotherapeutic agent that acts to suppress the proliferation of bacteria or kill bacteria.

The antibacterial agent according to the present invention may appropriately contain an optional ingredient used according to the type of the antibacterial agent within a range not impairing the effects of the present invention. Examples of the optional ingredient other than the antibacterial composition according to the present invention include an ingredient used for a cosmetic or a pharmaceutical, water, an oily ingredient, a thickener, a neutralizer, a surfactant, an emulsifier, an antiseptic, a bactericide, an antioxidant, a moisturizer, an ultraviolet absorber, a chelating agent, a pH adjuster, a skin-brightening agent, a blood circulation-promoter, a colorant, a propellant, an anti-inflammatory agent, a salt of a bioactive ingredient, a perfume, a plant extract, an analgesia antiphlogistic, a bactericidal disinfectant, a styptic, an emollient, a hormone preparation, and an antibiotic.

The form of the antibacterial agent according to the present invention may be, for example, a tablet, a capsule, a granule, a liquid, an emulsion, a cream, an ointment, a paste, a gel, a wax, or the like.

A method for producing the antibacterial agent according to the present invention is not particularly limited. For example, the antibacterial agent can be produced by stirring and mixing the ingredients with a publicly known device.

### [Cosmetic]

The antibacterial composition according to the present invention can be suitably used for a cosmetic. Specifically, a cosmetic of the present invention contains the antibacterial composition according to the present invention.

The cosmetic according to the present invention may appropriately contain an optional ingredient used according to the type of the cosmetic within a range not impairing the effects of the present invention. Examples of the optional ingredient other than the antibacterial composition according to the present invention include an ingredient used for a cosmetic or a pharmaceutical, water, an oily ingredient, a thickener, a neutralizer, a surfactant, an emulsifier, an antiseptic, a bactericide, an antioxidant, a moisturizer, an ultraviolet absorber, a chelating agent, a pH adjuster, a skin-brightening agent, a blood circulation-promoter, a colorant, a propellant, an anti-inflammatory agent, a salt of a bioactive ingredient, a perfume, and a plant extract.

The form of the cosmetic according to the present invention may be, for example, a liquid, an emulsion, a cream, an ointment, a paste, a gel, a wax, or the like.

The cosmetic of the present invention can be applied, for example, to the skin or the like, and can be preferably used in an application or spraying form.

A method for producing the cosmetic according to the present invention is not particularly limited. For example, the cosmetic can be produced by stirring and mixing the ingredients with a publicly known device.

### [Dermatological Topical Agent]

The antibacterial composition according to the present invention can be suitably used for a dermatological topical agent. Specifically, a dermatological topical agent of the present invention contains the antibacterial composition according to the present invention.

The dermatological topical agent according to the present invention may appropriately contain an optional ingredient used according to the type of the dermatological topical agent within a range not impairing the effects of the present invention. Examples of the optional ingredient other than the antibacterial composition according to the present invention include an ingredient used for a cosmetic or a pharmaceutical, water, an oily ingredient, a thickener, a neutralizer, a surfactant, an emulsifier, an antiseptic, a bactericide, an antioxidant, a moisturizer, an ultraviolet absorber, a chelating agent, a pH adjuster, a skin-brightening agent, a blood circulation-promoter, a colorant, a propellant, an anti-inflammatory agent, a salt of a bioactive ingredient, a perfume, a plant extract, an analgesia antiphlogistic, a bactericidal disinfectant, a styptic, an emollient, a hormone preparation, and an antibiotic.

The form of the dermatological topical agent according to the present invention may be, for example, a liquid, an emulsion, a cream, an ointment, a paste, a gel, a wax, or the like.

For example, the dermatological topical agent according to the present invention can be administered to the skin by applying or spraying.

The dermatological topical agent according to the present invention can be administered to the skin once or several times daily in an appropriate amount in accordance with the state or symptom of the skin and the form of the dermatological topical agent, by a method according to the form of the dermatological topical agent.

The dermatological topical agent according to the present invention is preferably used after bathing, face washing, housework using water, or the like.

According to the dermatological topical agent according to the present invention, Staphylococcus aureus is selectively inhibited as compared with Staphylococcus epidermidis that is indigenous bacteria on the skin, to achieve effects, such as prevention or improvement of drying of the skin, amelioration of cutaneous symptoms caused by atopic dermatitis, and prevention or improvement of a coarse skin.

A method for producing the dermatological topical agent according to the present invention is not particularly limited. For example, the dermatological topical agent can be produced by stirring and mixing the ingredients with a publicly known device.

### Examples

Hereinafter, the present invention will be specifically described by Examples, but the present invention is not limited to these Examples at all.

### Example 1

### (1) Production of Fermented Coconut Oil (Antibacterial Composition) by Fermentation of Coconut Oil using Staphylococcus Epidermidis

As a standard culture medium for activating Staphylococcus epidermidis, 899 g of aqueous solution containing casein peptone, a yeast extract, sodium chloride, and magnesium sulfate was prepared, and 100 g of coconut oil was added to this standard culture medium.

Subsequently, 1 g of surfactant was added to the solution to sufficiently bring the coconut oil into contact with the culture medium, and 6.09 LogCFU/mL of Staphylococcus epidermidis was inoculated with vigorous stirring such that an aqueous layer and an oil layer were mixed to obtain an emulsion liquid.

While the whole container was kept at 30°C and Staphylococcus epidermidis was sufficiently brought into contact with air, cultivation was then performed for a predetermined period of time until fermentation sufficiently proceeded.

After completion of the cultivation, an autoclave treatment was performed at 121°C until the solution reached a cloud point, resulting in demulsification at the same time of sterilization, to separate the solution into an aqueous layer and an oil layer. Subsequently, the solution was cooled in a refrigerator, and the separated oil layer was coagulated and taken out.

The taken oil layer was heated and melted, to obtain a white cloudy oil. This oil was separated by centrifugation into an aqueous layer containing the culture medium, a contaminant layer containing dead bacteria, and a transparent oil layer. The obtained transparent oily ingredient was called fermented coconut oil.

Herein, the following reagents were used in the production of the fermented coconut oil.
Coconut oil: Wako 1st Grade manufactured by FUJIFII,M Wako Pure Chemical Corporation
Staphylococcus epidermidis: Staphylococcus epidermidis ATCC 12228 manufactured by National Institute of Technology and Evaluation
Casein peptone: a microorganism cultivation base, HIPOLYPEPTON manufactured by NIHON PHARMACEUTICAL CO., LTD.
Yeast extract: Difco (trademark) Yeast Extract, UF, manufactured by Becton, Dickinson and Company
Sodium chloride: guaranteed reagent manufactured by FUJIFII,M Wako Pure Chemical Corporation
Magnesium sulfate: magnesium sulfate heptahydrate, guaranteed reagent manufactured by FUJIFII,M Wako Pure Chemical Corporation
Surfactant: polyoxyethylene (20) sorbitan monolaurate manufactured by FUJIFILM Wako Pure Chemical Corporation

### (2) Confirmation of Free Fatty Acid Group contained in Obtained Fermented Coconut Oil

The production of fermented coconut oil was performed three times by the method in (1) described above. The fatty acid concentrations of the respective fermented coconut oils were measured by an enzymatic colorimetric method (LabAssay (trademark) NEFA manufactured by FUJIFILM Wako Pure Chemical Corporation). As a result, the fatty acid concentrations of the fermented coconut oils were 978 mEq/L, 911 mEq/L, and 1,390 mEq/L.

These results confirmed that the fermented coconut oil obtained by fermentation of coconut oil using Staphylococcus epidermidis was an oily ingredient containing a fatty acid, that was, a composition containing a free fatty acid group.

The fermented coconut oil obtained was analyzed under the following condition by high performance liquid chromatography. As a result, the fermented coconut oil contained the following ingredients. Herein, the whole amount of the fermented coconut oil (antibacterial composition) was 100 mass%.
Caprylic acid (saturated fatty acid having 8 carbon atoms): 1.1 mass%
Capric acid (saturated fatty acid having 10 carbon atoms): 1.2 mass%
Lauric acid (saturated fatty acid having 12 carbon atoms): 7.9 mass%
Myristic acid (saturated fatty acid having 14 carbon atoms): 2.9 mass%
Palmitic acid (saturated fatty acid having 16 carbon atoms): 2.1 mass%
Stearic acid (saturated fatty acid having 18 carbon atoms): 0.4 mass%
Oleic acid (unsaturated fatty acid having 18 carbon atoms and an unsaturated bond): 1.1 mass%
Linoleic acid (unsaturated fatty acid having 18 carbon atoms and 2 unsaturated bonds): 0.2 mass%
Saturated or unsaturated fatty acid other than the saturated or unsaturated fatty acids, triacylglycerol, diacylglycerol, monoacylglycerol, and another contaminant: 83.1 mass% · Measurement condition of high performance liquid chromatography

A sample was warmed in a water bath of 40°C, to obtain a liquid, and the liquid was sufficiently shaken, to collect a homogeneous liquid. The liquid was diluted with an organic solvent, a derivatization reagent was added, and measurement was performed by high performance liquid chromatograph.

For caprylic acid (C8), capric acid (C10), myristic acid (C14), palmitic acid (C16), stearic acid (C18), oleic acid (C18:1), and linoleic acid (C18:2), a fluorescence detector was used, and for lauric acid (C12), a PDA detector was used.

### (3) Confirmation of Selective Antibacterial Properties of Obtained Fermented Coconut Oil

10% of the obtained fermented coconut oil was added to a culture medium for staphylococcus, and an emulsification treatment was performed. Staphylococcus epidermidis (was the same as Staphylococcus epidermidis used in (1)) and Staphylococcus aureus (NBRC13276 Staphylococcus aureus subsp. aureus Rosenbach 1884 manufactured by National Institute of Technology and Evaluation) were each inoculated and cultivated for 72 hours (called Staphylococcus epidermidis test area and Staphylococcus aureus test area, respectively).

The cultivated solution was diluted 10 times, applied to a standard agar medium (Nissui plate standard agar medium manufactured by Nissui Pharmaceutical Co., Ltd.), and cultivated for 72 hours.

As a result, a large number of colonies were observed at the Staphylococcus epidermidis test area (that was, Staphylococcus epidermidis lived), and a colony was not observed at the Staphylococcus aureus test area (that was, Staphylococcus aureus died), as shown in Fig. 1.

The result confirmed that the fermented coconut oil obtained by using Staphylococcus epidermidis had selective antibacterial properties in which the death of Staphylococcus epidermidis as useful bacteria was suppressed and Staphylococcus aureus as harmful bacteria was killed.

### Example 2

10 mass% of fermented coconut oil (free fatty acid concentration: 1,270 mEq/L) obtained by the same procedure as in (1) of Example 1 was added to a standard culture medium and emulsified by a supersonic treatment, to obtain a test culture medium.

Provided that to the standard medium, 0.1 mass% of polyoxyethylene sorbitan monolaurate (manufactured by FUJIFII,M Wako Pure Chemical Corporation) was added as an emulsifier.

Into the test culture medium, 6.61 LogCFU/mL of Staphylococcus aureus was inoculated and cultivated under shaking at 30°C for 48 hours.

The viable cell count of Staphylococcus aureus cultivated in the test culture medium was measured by a plate dilution method using a Mannitol salt agar with egg yolk. As a result, the viable cell count was less than 2 LogCFU/mL (negative).

### Comparative Example 1

The same operation as in Example 2 except that untreated coconut oil was used instead of fermented coconut oil having a free fatty acid concentration of 1,270 mEq/L was performed. As a result, the viable cell count was 8.2 LogCFU/mL.

### Comparative Examples 2 to 4

Free lauric acid (manufactured by FUJIFILM Wako Pure Chemical Corporation) was added to untreated coconut oil, to obtain an antibacterial composition having a free lauric acid concentration of 3.1 mass%, 6.3 mass%, or 12.5 mass%.

The same operation as in Example 2 except that the antibacterial composition having a free lauric acid concentration of 3.1 mass%, 6.3 mass%, or 12.5 mass% was used instead of fermented coconut oil obtained by the same procedure as in Example 2 was performed. As a result, the viable cell counts were 8.53 LogCFU/mL, 8.48 LogCFU/mL, and 8.20 LogCFU/mL. The free fatty acid concentration in Example 2 that was predicted from the measurement result of free fatty acid concentration by an enzymatic colorimetric method and the composition analysis result of free fatty acid by high performance liquid chromatography was about 11.9 mass%.

## Claims

1. A method for producing an antibacterial composition comprising: a step of producing a free fatty acid group by simple lipid and Staphylococcus epidermidis.

2. The method for producing an antibacterial composition according to claim 1,
wherein the simple lipid contains at least one selected from the group consisting of oil and fat and wax.

3. The method for producing an antibacterial composition according to claim 1 or 2,
wherein the simple lipid contains a saturated fatty acid having 8 or more and 18 or less carbon atoms in an amount of 10 mass% or more relative to 100 mass% of a total amount of a fatty acid moiety constituting the simple lipid.

4. The method for producing an antibacterial composition according to any of claims 1 to 3,
wherein the simple lipid contains at least one type of oil and fat selected from the group consisting of coconut oil, palm kernel oil, palm olein, palm oil, linseed oil, grape oil, soybean oil, sunflower oil, corn oil, cottonseed oil, sesame oil, rice oil, peanut oil, and olive oil.

5. The method for producing an antibacterial composition according to any of claims 1 to 4,
wherein a content of the free fatty acid having 8 or more and 18 or less carbon atoms in the antibacterial composition is 5 mass% or more and 50 mass% or less relative to 100 mass% of a total amount of the antibacterial composition.

6. An antibacterial composition obtained by the production method according to any of claims 1 to 5.

7. An antibacterial composition comprising: a free fatty acid group derived from Staphylococcus epidermidis.

8. The antibacterial composition according to claim 7,
wherein a content of the free fatty acid having 8 or more and 18 or less carbon atoms in the antibacterial composition is 5 mass% or more and 50 mass% or less relative to 100 mass% of a total amount of the antibacterial composition.

9. An antibacterial method comprising: a step of inhibiting Staphylococcus aureus by the antibacterial composition according to any of claims 6 to 8.

10. An antibacterial agent comprising: the antibacterial composition according to one of claims 6 to 8.

11. A cosmetic comprising: the antibacterial composition according to any of claims 6 to 8.

12. A dermatological topical agent comprising: the antibacterial composition according to any of claims 6 to 8.
